# EUROPEAN PATENT APPLICATION

(11) **EP 3 984 639 A1**
(43) Date of publication of application: **20.04.2022**
(21) Application number: 19933242.0
(22) Date of filing: 17.06.2019
(51) Int. Cl.: B01L 3/00, G01N 33/50

(54) **DETECTION CHIP AND PREPARATION METHOD THEREFOR**

(71) Applicant: BOE TECHNOLOGY GROUP CO., LTD., Beijing 100015 (CN)
(72) Inventor: YIN, Yudan, Beijing 100176 (CN)
(74) Representative: Brötz, Helmut
(86) International application number: PCT/CN2019/091555
(87) International publication number: WO 2020/252627

(57) **Abstract**

A detection chip and a method of manufacturing a detection chip are disclosed. The detection chip (100) includes a first substrate (110), a plurality of detection units (120), and at least one diversion dam (130). The plurality of detection units (120) are located on the first substrate (110), the diversion dam (130) is located on the first substrate (110), and the diversion dam (130) extends along a first path and is located between adjacent detection units (120). The detection chip (100) may improve the stability and parallelism of the flow field, facilitate improving the accuracy of the immunodetection result, and have the characteristics of small size, high throughput, and the like.

## Description

### TECHNICAL FIELD

Embodiments of the present disclosure relate to a detection chip and a method of manufacturing a detection chip.

### BACKGROUND

With development of biochemical detection technologies, the microfluidic chip has gradually become the research focus, and involves various subjects such as the biology, chemistry, medicine, fluid, electronics, materials, mechanics, and the like. The microfluidic chip can process and manipulate the micro-scale (e.g., 10⁻⁹ to 10⁻¹⁸ liters) fluid in channels of scales ranging from tens micrometers to hundreds of micrometers. The micro-scale structure of the microfluidic chip significantly increases the specific surface area of the fluid, that is, the ratio of surface area to volume of the fluid, thereby resulting in a series of surface-related special effects, such as the laminar flow effect, surface tension, capillary effect, rapid heat conduction effect, diffusion effect, etc. Therefore, the microfluidic chip has superior performance which the macro-scale laboratory device may not have, and has a broad application prospect.

### SUMMARY

At least one embodiment of the present disclosure provides a detection chip, and the detection chip includes: a first substrate; a plurality of detection units on the first substrate; and at least one diversion dam on the first substrate. The diversion dam extends along a first path and is between adjacent detection units.

For example, in the detection chip provided by an embodiment of the present disclosure, the plurality of detection units are arranged in a plurality of columns, and the first path extends along a column direction.

For example, in the detection chip provided by an embodiment of the present disclosure, the diversion dam is on both sides of each column of the detection units.

For example, in the detection chip provided by an embodiment of the present disclosure, the at least one diversion dam includes a plurality of diversion dams, and the plurality of diversion dams are parallel to each other.

For example, in the detection chip provided by an embodiment of the present disclosure, a height of the diversion dam in a direction perpendicular to the first substrate is greater than a height of the detection units in the direction perpendicular to the first substrate.

For example, the detection chip provided by an embodiment of the present disclosure further includes a hydrophilic layer, and the hydrophilic layer covers the detection units and the diversion dam.

For example, in the detection chip provided by an embodiment of the present disclosure, a portion of the hydrophilic layer covering the detection units includes a chemical modification group.

For example, the detection chip provided by an embodiment of the present disclosure further includes a hydrophobic layer, the hydrophobic layer is on the first substrate, and the detection units and the diversion dam are on the hydrophobic layer.

For example, the detection chip provided by an embodiment of the present disclosure further includes a second substrate, and the second substrate is opposite to the first substrate, and is separated from the first substrate to provide a detection space.

For example, the detection chip provided by an embodiment of the present disclosure further includes a sample inlet and a sample outlet, and the sample inlet and the sample outlet are on the second substrate.

For example, the detection chip provided by an embodiment of the present disclosure further includes a detection region, the plurality of detection units are in the detection region, the plurality of detection units are arranged in a plurality of columns, and the sample inlet and the sample outlet are distributed on different sides of the detection region along a column direction.

For example, in the detection chip provided by an embodiment of the present disclosure, the sample inlet and the sample outlet are distributed on different sides of the detection region in axial symmetry along the column direction or in central symmetry.

For example, in the detection chip provided by an embodiment of the present disclosure, the first substrate and/or the second substrate is a glass substrate.

For example, the detection chip provided by an embodiment of the present disclosure further includes a frame sealant, and the frame sealant is between the first substrate and the second substrate and around the diversion dam and the plurality of detection units.

For example, in the detection chip provided by an embodiment of the present disclosure, a height of the diversion dam is a ratio, which ranges from 30% to 60%, of a distance between the first substrate and the second substrate.

For example, in the detection chip provided by an embodiment of the present disclosure, a width of the diversion dam ranges from 50 microns to 200 microns, and a distance between the diversion dam and an adjacent detection unit is greater than or equal to 100 microns.

For example, in the detection chip provided by an embodiment of the present disclosure, a length of the diversion dam is greater than or equal to a sum of lengths of the plurality of detection units in the first path.

For example, in the detection chip provided by an embodiment of the present disclosure, a cross-sectional shape of the diversion dam in a direction perpendicular to the first path is a rectangle, a square, a trapezoid, or a semicircle.

For example, in the detection chip provided by an embodiment of the present disclosure, a material of the diversion dam includes photoresist.

For example, the detection chip provided by an embodiment of the present disclosure further includes a positioner, the positioner is on the first substrate, and the hydrophobic layer is on the positioner.

At least one embodiment of the present disclosure further provides a method of manufacturing the detection chip according to any one of the embodiments of the present disclosure, and the method includes: forming the plurality of detection units and the at least one diversion dam on the first substrate. The diversion dam extends along the first path and is between adjacent detection units.

### BRIEF DESCRIPTION OF THE DRAWINGS

In order to clearly illustrate the technical solution of the embodiments of the present disclosure, the drawings of the embodiments will be briefly described in the following. It is obvious that the described drawings in the following are only related to some embodiments of the present disclosure and thus are not limitative of the present disclosure.
FIG 1 is a schematic planar diagram of a detection chip provided by some embodiments of the present disclosure;
FIG 2 is a schematic cross-sectional diagram of the detection chip illustrated in FIG. 1;
FIG. 3 is a schematic diagram of flow field simulation of a detection chip provided by some embodiments of the present disclosure;
FIG. 4 is a schematic planar diagram of another detection chip provided by some embodiments of the present disclosure;
FIG. 5 is a schematic cross-sectional diagram of the detection chip illustrated in FIG. 4; and
FIG. 6 is a schematic flowchart of a method of manufacturing a detection chip provided by some embodiments of the present disclosure.

### DETAILED DESCRIPTION

In order to make objects, technical details and advantages of the embodiments of the disclosure apparent, the technical solutions of the embodiments will be described in a clearly and fully understandable way in connection with the drawings related to the embodiments of the disclosure. Apparently, the described embodiments are just a part but not all of the embodiments of the disclosure. Based on the described embodiments herein, those skilled in the art can obtain other embodiment(s), without any inventive work, which should be within the scope of the disclosure.

Unless otherwise defined, all the technical and scientific terms used herein have the same meanings as commonly understood by one of ordinary skill in the art to which the present disclosure belongs. The terms "first," "second," etc., which are used in the description and the claims of the present application for disclosure, are not intended to indicate any sequence, amount or importance, but distinguish various components. Also, the terms "comprise," "comprising," "include," "including," etc., are intended to specify that the elements or the objects stated before these terms encompass the elements or the objects and equivalents thereof listed after these terms, but do not preclude the other elements or objects. The phrases "connect", "connected", "coupled", etc., are not intended to define a physical connection or mechanical connection, but may include an electrical connection, directly or indirectly. "On," "under," "right," "left" and the like are only used to indicate relative position relationship, and when the position of the object which is described is changed, the relative position relationship may be changed accordingly.

The immunodetection chip is a commonly used microfluidic chip and can be used to detect cancer biomarkers, heart biomarkers, thyroid biomarkers, gonad biomarkers, and other infectious disease biomarkers. For example, the target antigen or antibody may be attached to the detection units in the immunodetection chip, then the immunodetection chip is encapsulated, and the solution to be detected is injected into the immunodetection chip to allow the solution to be detected to flow through the region where the detection units are located. In the case where there are biomarkers in the solution to be detected, these biomarkers may bind to the antigen or antibody attached to the detection units while the solution to be detected flows, so that the biomarkers are fixed to the detection units. Then, the detection units are subjected to the optical detection (for example, fluorescence detection), so that the immunodetection result may be obtained.

However, because the internal space of the immunodetection chip is in the micron scale, the injection speed, the injection volume, the temperature and other factors of the solution to be detected have a great influence on the flow field in the chip, and therefore, the stability and parallelism of the flow field in the chip may be poor, which may be adverse to the binding of the biomarkers in the solution to be detected and the target antigen or antibody on the detection units, thereby adversely affecting the immunodetection result.

At least one embodiment of the present disclosure provides a detection chip and a method of manufacturing a detection chip, and the detection chip can improve the stability and parallelism of the flow field, facilitate improving the accuracy of the immunodetection result, and have the characteristics of small size, high throughput, and the like.

Hereinafter, the embodiments of the present disclosure are described in detail with reference to the accompanying drawings. It should be noted that the same reference numerals in different drawings are used to refer to the same described components or elements.

At least one embodiment of the present disclosure provides a detection chip, and the detection chip includes a first substrate, a plurality of detection units, and at least one diversion dam. The plurality of detection units are located on the first substrate, the diversion dam is located on the first substrate, and the diversion dam extends along a first path and is located between adjacent detection units.

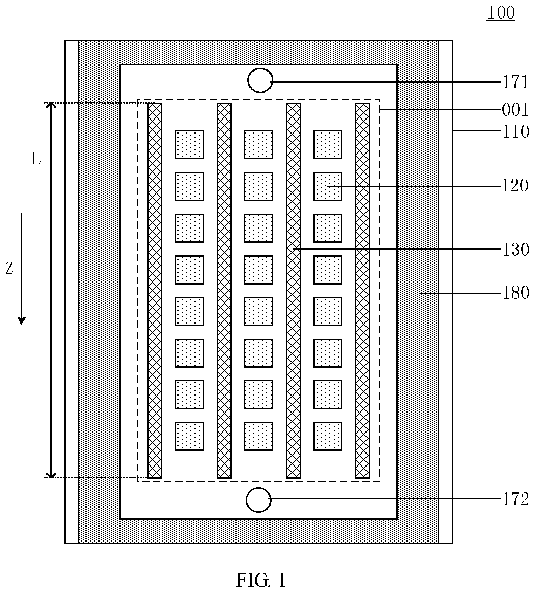

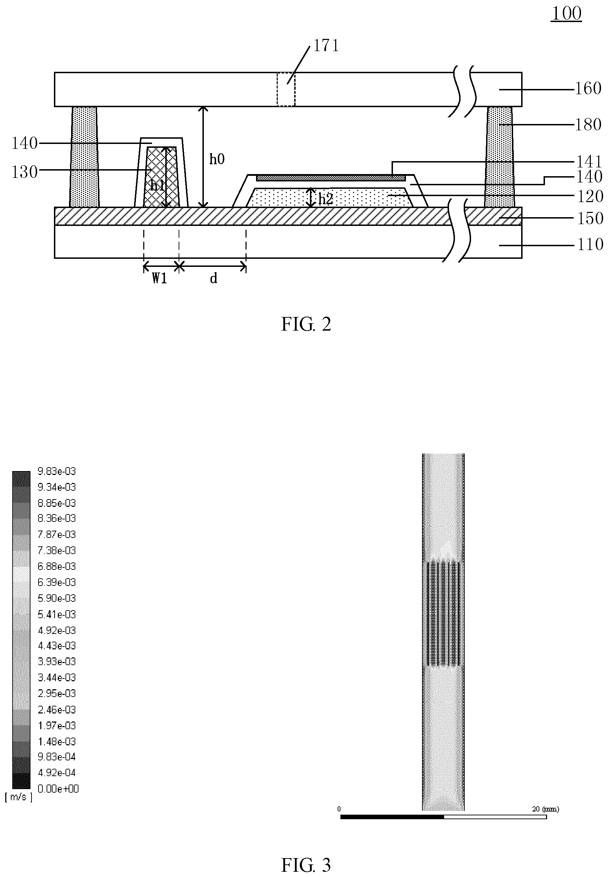

FIG. 1 is a schematic planar diagram of a detection chip provided by some embodiments of the present disclosure, and FIG. 2 is a schematic cross-sectional diagram of the detection chip illustrated in FIG. 1. As illustrated in FIG. 1 and FIG. 2, a detection chip 100 includes a first substrate 110, a plurality of detection units 120, and at least one diversion dam 130.

The first substrate 110 provides functions such as support, protection, and the like, and may be a plastic substrate, a glass substrate, a silicon substrate, or other suitable substrates, which is not limited in the embodiments of the present disclosure. For example, in the case where a glass substrate is adopted, the cost is lower; and in the case where a silicon substrate is adopted, the performance is better. For example, the first substrate 110 is a transparent substrate (such as a glass substrate), so that light can pass through the transparent substrate without loss or with less loss, thereby improving the accuracy of subsequent optical detection and lowering the requirements for the optical detection equipment additionally provided.

The plurality of detection units 120 are located on the first substrate 110, and the detection units 120 are used to, for example, provide attachment locations for target antigens or antibodies. For example, in some examples, the detection unit 120 is in the shape of a boss, thereby facilitating the binding or reaction of the target antigens or antibodies attached to the detection unit 120 and the biomarkers in the solution to be detected flowing through the detection unit 120. Of course, the embodiments of the present disclosure are not limited to this case, and the detection unit 120 may also have a groove shape or a planar shape, as long as it may be ensured that the target antigens or antibodies attached to the detection unit 120 can contact the solution to be detected flowing through the detection unit 120 and can be combined with the biomarkers in the solution. It should be noted that, in the embodiments of the present disclosure, the number of the detection units 120 is not limited, and may be any value, and for example, the number may be determined according to the type or concentration of the biomarkers to be detected.

The diversion dam 130 is located on the first substrate 110, and the diversion dam 130 extends along a first path and is located between adjacent detection units 120. The diversion dam 130 affects the flow field in the internal space of the detection chip 100, thereby improving the uniformity of the flow velocities at the locations of different detection units 120, improving the parallelism of the flow field along the first path, and improving the stability of the flow field, so as to allow the solution to be detected to flow stably and uniformly through the region where the detection units 120 are located. Therefore, the biomarkers in the solution to be detected may sufficiently bind or react with the target antigens or antibodies on the detection units 120, thereby facilitating improving the accuracy and reliability of the immunodetection result. Moreover, the detection chip 100 also has the characteristics of small size, high throughput, etc.

For example, in some examples, as illustrated in FIG. 1, the plurality of detection units 120 are arranged in a plurality of columns, and the first path extends along a column direction Z. The diversion dams 130 are provided on both sides of each column of the detection units 120, and the plurality of diversion dams 130 are parallel to each other. In the case where the solution to be detected flows through the plurality of detection units 120 along the column direction Z, under the action of the diversion dams 130, the parallelism of the flow field formed by the flow of the solution to be detected along the column direction Z is improved, so that the solution to be detected may flow stably and uniformly along the column direction Z.

It should be noted that in the embodiments of the present disclosure, the first path is not limited to extending in the column direction Z, but may also extend in any other direction. In addition, the first path may extend along a straight line or a curve, which may be determined according to the flow path and flow manner of the solution to be detected, and the embodiments of the present disclosure are not limited in this aspect. For example, in the case where the first path extends along a straight line, the diversion dam 130 also extends along a straight line; and in the case where the first path extends along a curve, the diversion dam 130 also extends along a curve. Correspondingly, the plurality of detection units 120 may be arranged in a plurality of columns along a straight line, or may be arranged in a plurality of groups along a curve, and the diversion dam 130 located between the adjacent detection units 120 only needs to extend along the arrangement direction of the detection units 120.

It should be noted that, in the embodiments of the present disclosure, the diversion dam 130 may be provided on both sides of each column of the detection units 120, or only provided on both sides of some columns of the detection units 120, which depends on the parallelism of the flow field to be achieved, and the embodiments of the present disclosure are not limited in this aspect.

For example, in the case where the diversion dam 130 is provided on both sides of each column of the detection units 120 (for example, in the case where the detection units 120 and the diversion dams 130 are provided as illustrated in FIG. 1), the flow field has good parallelism. In the case where the target antigens or antibodies on one detection unit 120 accidentally drop, the dropped target antigens or antibodies may flow along the column direction Z, that is, flow in the region where this column of detection units 120 is located, so other columns of detection units 120 may not be affected, thereby avoiding the crosstalk between different detection points (i.e., different detection units 120) and avoiding cross contamination.

For example, the number of the diversion dams 130 is not limited, and the diversion dam 130 may be one or the diversion dams 130 may be multiple. For example, in some examples, in the case where the plurality of detection units 120 are arranged in only two columns, only one diversion dam 130 may be provided, and the diversion dam 130 is located between the two columns of detection units 120, thereby allowing the flow field to have better parallelism in the case of reducing the number of the diversion dams 130.

For example, the cross-sectional shape of the diversion dam 130 in a direction perpendicular to the first path (e.g., the column direction Z) may be a rectangle, a square, a trapezoid, a semicircle, or other suitable shapes, such as a regular shape or an irregular shape, which is not limited in the embodiments of the present disclosure. For example, different cross-sectional shapes have different effects on the flow field, and therefore, the cross-sectional shape of the diversion dam 130 may be determined according to the characteristics of the flow field.

For example, both the diversion dam 130 and the detection unit 120 may be manufactured by using photoresist, and for example, the photoresist may be thick film etchable photoresist. For example, in some examples, the diversion dam 130 and the detection unit 120 may be formed in the same patterning process to simplify the production process.

For example, as illustrated in FIG. 2, in some examples, the detection chip 100 further includes a hydrophilic layer 140, and the hydrophilic layer 140 covers the detection unit 120 and the diversion dam 130. By providing the hydrophilic layer 140, the solution to be detected may better contact with the detection unit 120, thereby facilitating the binding of the biomarkers in the solution to be detected and the target antigens or antibodies on the detection unit 120.

For example, the material of the hydrophilic layer 140 is silicon oxide, such as silicon dioxide (SiO2) or the like. Of course, the embodiments of the present disclosure are not limited thereto, and the hydrophilic layer 140 may also be prepared by using other suitable inorganic or organic materials, as long as the surface of the hydrophilic layer 140 in contact with the solution to be detected is hydrophilic. For example, the hydrophilic layer 140 may be directly prepared by using a hydrophilic material. For another example, the hydrophilic layer 140 may be made of a material which does not have hydrophilicity, and in this case, the hydrophilic layer 140 needs to be subjected to a hydrophilic treatment on the surface in contact with the solution to be detected, so as to allow the hydrophilic layer 140 to have hydrophilicity. For example, in the case where a non-hydrophilic material, such as silicon nitride or the like, is used, the non-hydrophilic material may be subjected to a hydrophilic treatment, for example, by using a gelation modification method, an ultraviolet radiation method, a plasma method, or the like, and for example, the surface of the non-hydrophilic material may be provided with the hydrophilic group, so as to allow the non-hydrophilic material to have hydrophilicity.

For example, as illustrated in FIG. 2, in some examples, the portion of the hydrophilic layer 140 covering the detection unit 120 includes a chemical modification group 141. For example, the chemical modification group 141 may be obtained through chemical modification treatment, and may be combined with the target antigens or antibodies. By providing the chemical modification group 141, the target antigens or antibodies can be stably attached to the detection unit 120, and the target antigens or antibodies may not easily fall off under the flow of the solution to be detected, thereby improving the accuracy of the immunodetection result.

For example, as illustrated in FIG. 2, in some examples, the detection chip 100 further includes a hydrophobic layer 150. The hydrophobic layer 150 is provided on the first substrate 110, and the detection unit 120 and the diversion dam 130 are provided on the hydrophobic layer 150. By providing the hydrophobic layer 150, the solution to be detected may flow more easily in the detection chip 100, and the biomarkers in the solution to be detected may not be easily attached to the first substrate 110, so as to avoid the waste of the biomarkers in the solution to be detected.

For example, the material of the hydrophobic layer 150 is resin or silicon nitride. Of course, the hydrophobic layer 150 may also be made of other suitable inorganic or organic materials, as long as the surface of the hydrophobic layer 150 away from the first substrate 110 is hydrophobic. For example, the hydrophobic layer 150 may be directly prepared by using a hydrophobic material. For another example, the hydrophobic layer 150 may be made of a material which does not have hydrophobicity, and in this case, the surface of the hydrophobic layer 150 away from the first substrate 110 needs to be subjected to a hydrophobic treatment, so as to allow the surface of the hydrophobic layer 150 away from the first substrate 110 to have hydrophobicity.

For example, as illustrated in FIG. 2, in some examples, the detection chip 100 further includes a second substrate 160. For example, the second substrate 160 is opposite to the first substrate 110, and is separated from the first substrate 110 to provide a detection space (i.e., a liquid flowing space). The material of the second substrate 160 may be the same as or different from that of the first substrate 110, which is not limited in the embodiments of the present disclosure. For example, the second substrate 160 is a transparent substrate (such as a glass substrate), so that light may pass through the transparent substrate without loss or with less loss, thereby improving the accuracy of subsequent optical detection and lowering the requirements for the optical detection equipment additionally provided.

For example, as illustrated in FIG. 1, in some examples, the detection chip 100 further includes a sample inlet 171, a sample outlet 172, and a detection region 001. For example, the plurality of detection units 120 are located in the detection region 001, the plurality of detection units 120 are arranged in a plurality of columns, and the sample inlet 171 and the sample outlet 172 are distributed on different sides (for example, on the upside and downside in the figure) of the detection region 001 along the column direction Z. For example, the solution to be detected may be injected into the sample inlet 171 through a micro-injection pump or a pipette, and flow out from the sample outlet 172 after flowing through the plurality of detection units 120 along the column direction Z. For example, the sample inlet 171 and the sample outlet 172 are distributed on different sides of the detection region 001 in axial symmetry along the column direction Z or in central symmetry, so that the parallelism and stability of the flow field may be further improved. Of course, the embodiments of the present disclosure are not limited thereto, and the sample inlet 171 and the sample outlet 172 may also be distributed asymmetrically, which may be determined according to the characteristics of the flow field and actual requirements.

For example, the sample inlet 171 and the sample outlet 172 are provided on the second substrate 160. For example, as illustrated in FIG. 2, the sample inlet 171 may be a through hole penetrating the second substrate 160, and the shape of the through hole in the cross section parallel to the second substrate 160 may be a circle, a rectangle, a square, or any other suitable shapes. Similarly, the sample outlet 172 may also be a through hole penetrating the second substrate 160, and the shape of the sample outlet 172 in the cross section parallel to the second substrate 160 may be the same as or different from that of the sample inlet 171. It should be noted that FIG. 2 only schematically illustrates the arrangement of the sample inlet 171 on the second substrate 160, but the relative position of the sample inlet 171 and the detection unit 120 is not limited by the case illustrated in FIG. 2.

For example, as illustrated in FIG. 1 and FIG. 2, in some examples, the detection chip 100 further includes a frame sealant 180, and the frame sealant 180 is between the first substrate 110 and the second substrate 160 and around the diversion dam 130 and the plurality of detection units 120. For example, the first substrate 110, the second substrate 160, and the frame sealant 180 cooperate to define the flowing space of the solution to be detected. For example, in some examples, spacers may be mixed in the frame sealant 180, so that the distance between the first substrate 110 and the second substrate 160 may be controlled by the spacers, and the compressive strength of the detection chip 100 may be improved.

For example, as illustrated in FIG. 2, the height h1 of the diversion dam 130 in the direction perpendicular to the first substrate 110 is greater than the height h2 of the detection unit 120 in the direction perpendicular to the first substrate 110, so that the parallelism of the flow filed can be better adjusted. For example, the height h1 of the diversion dam 130 is a ratio, which ranges from 30% to 60%, of the distance h0 between the first substrate 110 and the second substrate 160, for example, 40% or 50%. For example, in some examples, the distance h0 between the first substrate 110 and the second substrate 160 is 100 µm, the height h1 of the diversion dam 130 is 50 µm, and the height h2 of the detection unit 120 is 3 µm, so that the height difference between h1 and h2 is large, which may better adjust the parallelism of the flow field. For example, in some examples, in the case where the cross-sectional shape of the diversion dam 130 in the direction perpendicular to the first path (e.g., the column direction Z) is a semicircle, the radius of the semicircle may be greater than or equal to a half of the distance h0 between the first substrate 110 and the second substrate 160.

For example, as illustrated in FIG. 2, the width W1 of the diversion dam 130 is in a range of 50 µm to 200 µm, such as 80 µm, 100 µm, or 150 µm. The distance d between the diversion dam 130 and the adjacent detection unit 120 is greater than or equal to 100 µm.

For example, as illustrated in FIG. 1, the length L of the diversion dam 130 is greater than or equal to a sum of lengths of the plurality of detection units 120 in the first path (e.g., the column direction Z). For example, in some examples, the length L of the diversion dam 130 is 1 cm.

It should be noted that in the embodiments of the present disclosure, the height h1 may refer to the height of the diversion dam 130 itself, and may also refer to the sum of the height of the diversion dam 130 and the height of the hydrophilic layer 140. Similarly, the height h2 may refer to the height of the detection unit 120 itself, and may also refer to the sum of the height of the detection unit 120 and the height of the hydrophilic layer 140. Similarly, the width W1 and the distance d may also take the thickness of the hydrophilic layer 140 into consideration.

When the detection chip 100 is used, the target antigens or antibodies are first attached to the detection units 120 before the first substrate 110 and the second substrate 160 are bonded to form a cell. For example, the liquid including the target antigens or antibodies may be dropped on the detection units 120, and because there are the chemical modification groups 141, the target antigens or antibodies bind to the chemical modification groups 141, so as to be attached to the detection units 120. Then, the first substrate 110 and the second substrate 160 are bonded to form a cell by using the frame sealant 180. Next, the solution to be detected is injected from the sample inlet 171, so that the solution to be detected flows through the detection region 001 and flows out from the sample outlet 172. When the biomarkers in the solution to be detected flow through the detection units 120, the biomarkers may bind or react with the target antigens or antibodies attached to the detection units 120. Then, for example, the bovine serum albumin (BSA) solution may be injected into the detection chip 100 to clean the internal space of the detection chip 100, so as to reduce the adsorption of the solution to be detected on the portion of the internal space of the detection chip 100 other than the detection units 120, thereby improving the accuracy of subsequent detection. Finally, the optical detection equipment is used to perform optical detection on the detection chip 100, so as to obtain the immunodetection result.

FIG. 3 is a schematic diagram of flow field simulation of a detection chip provided by some embodiments of the present disclosure, and for example, the simulation result is the simulation result of the detection chip 100 illustrated in FIG. 1 and FIG. 2. It can be seen from FIG. 3 that the uniformity of the flow field distribution in different regions within the detection chip 100 is relatively good because of the existence of the diversion dam 130. In addition, in the detection region 001 of the detection chip 100, the flow velocity in the region between the diversion dams 130 is large, so that the flow field has good stability and parallelism, thereby facilitating improving the accuracy of the immunodetection result. Moreover, it can be seen from FIG. 3 that the flow velocity in the region adjacent to the diversion dam 130 and located on both sides of the diversion dam 130 is relatively small, and therefore, it is necessary to maintain a certain distance (for example, the above-mentioned distance d, and d may be greater than or equal to 100 µm) between the diversion dam 130 and the detection unit 120, so as to allow the detection unit 120 to avoid the region where the flow velocity is small.

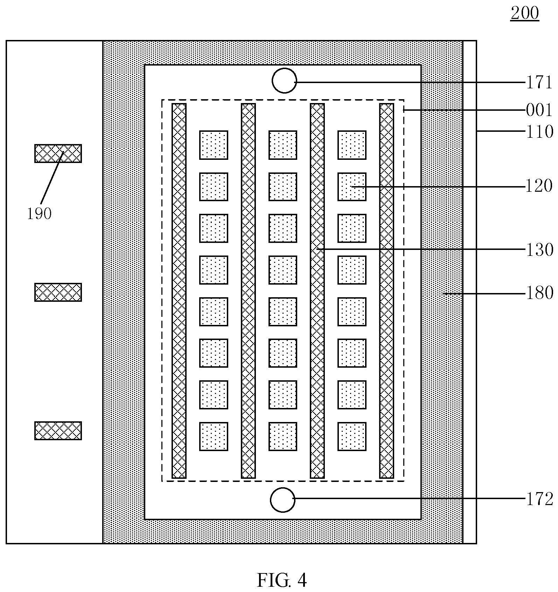

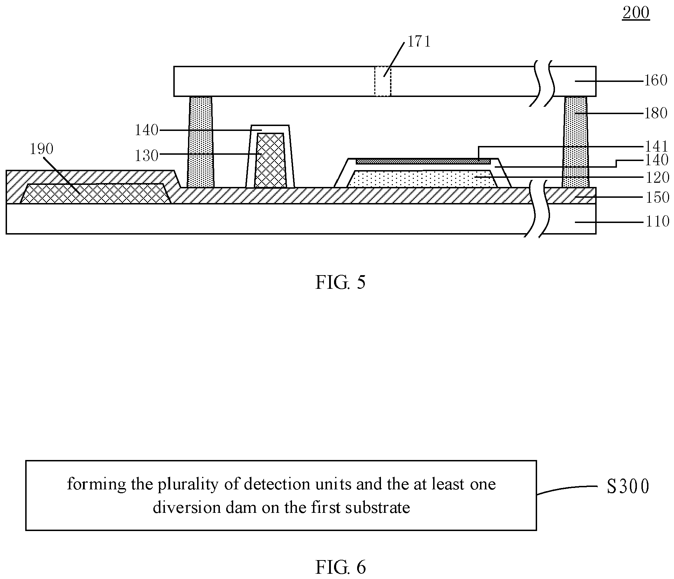

FIG. 4 is a schematic planar diagram of another detection chip provided by some embodiments of the present disclosure, and FIG. 5 is a schematic cross-sectional diagram of the detection chip illustrated in FIG. 4. As illustrated in FIG. 4 and FIG. 5, the detection chip 200 of this embodiment is basically the same as the detection chip 100 illustrated in FIG. 1 and FIG. 2 except that the positioner 190 is further included.

In this embodiment, the positioner 190 is used to cooperate with the optical detection equipment additionally provided to realize the positioning of the detection chip 200, thereby facilitating the optical detection equipment to perform optical detection on the detection chip 200. For example, the positioner 190 is provided on the first substrate 110 and is covered by the hydrophobic layer 150. The positioner 190 may be made of a metal material, such as molybdenum (Mo), or may be made of an opaque insulating material, which is not limited in the embodiments of the present disclosure. For example, in some examples, when the positioning is performed, the optical positioning device of the optical detection equipment emits light for positioning. In the case where the detection chip 200 is located at the predetermined position, because the positioner 190 cannot transmit light, the light intensity detected by the sensor provided at the corresponding position is very small or is zero, so that it can be determined that the detection chip 200 is at the predetermined position, so as to achieve positioning. After the positioning is completed, the optical detection equipment may be used to perform optical detection on the specific points and to perform signal reading. For example, the specific point may be a certain detection unit 120 or the specific points may be some detection units 120 where the target antigens or antibodies are attached.

For example, the positioner 190 is located outside the detection region 001, for example, further outside the liquid flowing space formed by the first substrate 110, the second substrate 160, and the frame sealant 180, so as not to affect the optical detection. For example, in some examples, as illustrated in FIG. 4, a plurality of positioners 190 are provided on one side of the detection chip 200 and close to the edge of the detection chip 200. By providing the plurality of positioners 190, the positioning accuracy may be improved. Of course, the embodiments of the present disclosure are not limited thereto, and the position of the positioner 190 may be determined according to actual needs, and for example, the positioner 190 may be provided on any one side, on any two sides, around, or at other suitable positions of the detection chip 200, which may be determined according to the positioning method of the cooperating optical detection equipment. The number of positioners 190 is also not limited, and may be any value, which may be determined according to actual needs.

At least one embodiment of the present disclosure further provides a method of manufacturing a detection chip, and the method can be used to manufacture the detection chip described in any one of the embodiments of the present disclosure. By using the method, the stability and parallelism of the flow field inside the detection chip can be improved, which facilitates improving the accuracy of the immunodetection result, and the manufactured detection chip has the characteristics of small size, high throughput, and the like.

FIG. 6 is a schematic flowchart of a method of manufacturing a detection chip provided by some embodiments of the present disclosure. For example, in some examples, as illustrated in FIG. 6, the method includes following operations.

Step S300: forming a plurality of detection units 120 and at least one diversion dam 130 on a first substrate 110, where the diversion dam 130 extends along a first path and is between adjacent detection units 120.

For example, the detection unit 120 and the diversion dam 130 may be formed by adopting the photoresist and using processes such as exposure, development, etching, and the like.

It should be noted that, in the embodiments of the present disclosure, the method may further include more steps, and the sequence of the steps is not limited, and may be determined according to actual needs. The detailed descriptions and technical effects of the method may be with reference to the above descriptions related to the detection chip 100/200, and details are not described herein again.

The following statements should be noted.
(1) The accompanying drawings involve only the structure(s) in connection with the embodiment(s) of the present disclosure, and other structure(s) can be referred to common design(s).
(2) In case of no conflict, features in one embodiment or in different embodiments can be combined to obtain new embodiments.

What have been described above are only specific implementations of the present disclosure, the protection scope of the present disclosure is not limited thereto, and the protection scope of the present disclosure should be based on the protection scope of the claims.

## Claims

1. A detection chip, comprising:
a first substrate;
a plurality of detection units on the first substrate; and
at least one diversion dam on the first substrate, the diversion dam extending along a first path and being between adjacent detection units.

2. The detection chip according to claim 1, wherein the plurality of detection units are arranged in a plurality of columns, and the first path extends along a column direction.

3. The detection chip according to claim 2, wherein the diversion dam is on both sides of each column of the detection units.

4. The detection chip according to any one of claims 1 to 3, wherein the at least one diversion dam comprises a plurality of diversion dams, and the plurality of diversion dams are parallel to each other.

5. The detection chip according to any one of claims 1 to 4, wherein a height of the diversion dam in a direction perpendicular to the first substrate is greater than a height of the detection units in the direction perpendicular to the first substrate.

6. The detection chip according to any one of claims 1 to 5, further comprising a hydrophilic layer, wherein the hydrophilic layer covers the detection units and the diversion dam.

7. The detection chip according to claim 6, wherein a portion of the hydrophilic layer covering the detection units comprises a chemical modification group.

8. The detection chip according to any one of claims 1 to 5, further comprising a hydrophobic layer, wherein the hydrophobic layer is on the first substrate, and the detection units and the diversion dam are on the hydrophobic layer.

9. The detection chip according to any one of claims 1 to 5, further comprising a second substrate, wherein the second substrate is opposite to the first substrate, and is separated from the first substrate to provide a detection space.

10. The detection chip according to claim 9, further comprising a sample inlet and a sample outlet, wherein the sample inlet and the sample outlet are on the second substrate.

11. The detection chip according to claim 10, further comprising a detection region, wherein the plurality of detection units are in the detection region,
the plurality of detection units are arranged in a plurality of columns, and the sample inlet and the sample outlet are distributed on different sides of the detection region along a column direction.

12. The detection chip according to claim 11, wherein the sample inlet and the sample outlet are distributed on different sides of the detection region in axial symmetry along the column direction or in central symmetry.

13. The detection chip according to any one of claims 9 to 12, wherein the first substrate and/or the second substrate is a glass substrate.

14. The detection chip according to any one of claims 9 to 13, further comprising a frame sealant, wherein the frame sealant is between the first substrate and the second substrate and around the diversion dam and the plurality of detection units.

15. The detection chip according to any one of claims 9 to 14, wherein a height of the diversion dam is a ratio, which ranges from 30% to 60%, of a distance between the first substrate and the second substrate.

16. The detection chip according to any one of claims 1 to 15, wherein a width of the diversion dam ranges from 50 microns to 200 microns, and a distance between the diversion dam and an adjacent detection unit is greater than or equal to 100 microns.

17. The detection chip according to any one of claims 1 to 16, wherein a length of the diversion dam is greater than or equal to a sum of lengths of the plurality of detection units in the first path.

18. The detection chip according to any one of claims 1 to 17, wherein a cross-sectional shape of the diversion dam in a direction perpendicular to the first path is a rectangle, a square, a trapezoid, or a semicircle.

19. The detection chip according to any one of claims 1 to 18, wherein a material of the diversion dam comprises photoresist.

20. The detection chip according to claim 8, further comprising a positioner, wherein the positioner is on the first substrate, and the hydrophobic layer is on the positioner.

21. A method of manufacturing the detection chip according to any one of claims 1 to 20, comprising:
forming the plurality of detection units and the at least one diversion dam on the first substrate,
wherein the diversion dam extends along the first path and is between adjacent detection units.
